# EUROPEAN PATENT APPLICATION

(11) **EP 4 650 459 A1**
(43) Date of publication of application: **19.11.2025**
(21) Application number: 24382534.6
(22) Date of filing: 17.05.2024
(51) Int. Cl.: C12Q 1/6883

(54) **PROGNOSIS OF A RESPIRATORY SYNCYTIAL VIRUS INFECTION**

(71) Applicant: Fundación Pública Galega Instituto de Investigación Sanitaria de Santiago de Compostela, 15706 Santiago de Compostela A Coruña (ES); Servizo Galego De Saúde, 15703 Santiago de Compostela, A Coruna (ES); Universidade de Santiago de Compostela, 15782 Santiago de Compostela (ES)
(72) Inventor: GÓMEZ CARBALLA, Alberto, 15706 Santiago de Compostela (ES); PISCHEDDA, Sara, 15706 Santiago de Compostela (ES); MARTINÓN TORRES, Federico, 15706 Santiago de Compostela (ES); SALAS ELLACURIAGA, Antonio, 15706 Santiago de Compostela (ES)
(74) Representative: Hoffmann Eitle

(57) **Abstract**

The present invention refers to an *in vitro* method for the prognosis of a Respiratory Syncytial Virus (RSV) infection, or for differentiating patients suffering from a severe RSV infection from patients that have a mild or moderate RSV.

## Description

### FIELD OF THE INVENTION

The present invention refers to the medical field. Particularly, it refers to an *in vitro* method for the prognosis of a Respiratory Syncytial Virus (RSV) infection, or for differentiating patients suffering from a severe RSV infection from patients that have a mild or moderate RSV.

### STATE OF THE ART

RSV, the primary cause of acute lower respiratory infections (ALRI) in young children across the globe, infects almost all children by the age of two and it is responsible for a considerable number of infant hospitalizations. Although most RSV hospitalizations occur in previously healthy children born at term, current prevention strategies still primarily focus on specific at-risk children. Evidence suggests that the severity of RSV bronchiolitis occurring in infancy could be associated with immunological mechanisms. In fact, RSV infections appear with more frequency in the first years of life when the immune system is immature. Severe RSV infections have been found to exhibit an inefficient activation of innate immunity and an acquired immune response activation bias toward T helper (Th) 2 and/or Th17 phenotypes. The excessive Th2 response is characterized by an overproduction of certain cytokines, which are signaling molecules that help to regulate the immune response. This can further exacerbate the respiratory symptoms experienced by infants with severe RSV infections.

It is important to understand the underlying molecular mechanisms of RSV infections and their immune response in order to develop more effective treatments and preventive measures. Targeting the specific pathways and cells involved in the immune response may reduce the severity of RSV infections and improve outcomes, especially in those patients with severe symptoms.

So, there is an unmet medical need of identifying reliable methods for prognosis of RSV infection, or for differentiating patients suffering from a severe RSV infection from patients that have a mild or moderate RSV. The present invention is focused on solving this problem and an innovative strategy for the prognosis of RSV infection, or for differentiating patients suffering from a severe RSV infection from patients that have a mild or moderate RSV, is herein provided.

### DESCRIPTION OF THE INVENTION

The present invention refers to a method for the prognosis of an RSV infection, or for differentiating patients suffering from a severe RSV infection from patients that have a mild or moderate RSV infection.

The present invention was developed bearing in mind that there is no previous evidence on how epigenetics mechanisms influence the different disease severity observed after RSV infection. Moreover, the use of saliva, due to its non-invasive nature, is convenient and preferred, particularly for methodologies involving children. Saliva is an attractive accessible source of cells from which a high quantity and quality of DNA/RNA can be obtained. No studies have explored saliva biomarkers associated with RSV disease or using epigenomics in this context. Consequently, the inventors of the present invention aimed at investigating the role of host epigenetics in the RSV severity infection using saliva samples collected from patients with mild/moderate and severe symptoms during the acute phase of the infection. To the best of our knowledge, this is the first-time epigenetics has been explored to study RSV severity in saliva samples. So, the technical feature which confers unity of invention is precisely the use of epigenetics to study RSV severity, particularly in saliva samples.

Thus, since RSV poses significant morbidity and mortality risk in childhood, particularly for previously healthy infants admitted to hospitals lacking predisposing risk factors for severe disease, the inventors of the present invention investigated the host epigenome's role in RSV infection severity using non-invasive saliva samples from sixteen hospitalized infants, eight with severe and eight presented mild to moderate symptoms. Epigenetic analyses used the Illumina EPIC BeadChip for DNA isolated from saliva samples. A panel of differentially methylated positions distinguishing severe from mild to moderate symptoms in infants was identified. Differentially methylated regions were identified in *ZBTB38* and *TRIM6-TRM34* genes.

The present invention provides biomarkers related to RSV infection and severity and shed further light on the local mechanisms that might be contributing to the severe phenotype, representing a good proof of principle for non-invasive saliva samples.

So, the first embodiment of the present invention refers to an *in vitro* method for the prognosis of a RSV infection, which comprises determining the methylation status of a gene selected from the group consisting of: *ZBTB38* and/or *TRIM6-TRIM34,* in a biological sample obtained from the patient; or to the *in vitro* use of the methylation status of a gene selected from the group consisting of: *ZBTB38* and/or *TRIM6-TRIM34,* or of a kit comprising reagents for determining the methylation status of a gene selected from the group consisting of: *ZBTB38* and/or *TRIM6-TRIM34,* for the prognosis of a RSV infection.

In a preferred embodiment, the present invention refers to an *in vitro* method for differentiating patients suffering a severe RSV infection from patients that have a mild or moderate RSV infection, which comprises determining the methylation status of the gene selected from the group consisting of: *ZBTB38* and/or *TRIM6-TRIM34,* in a biological sample obtained from the patient; or to the *in vitro* use of the methylation status of a gene selected from the group consisting of: *ZBTB38* and/or *TRIM6-TRIM34,* or of a kit comprising reagents for determining the methylation status of a gene selected from the group consisting of: *ZBTB38* and/or *TRIM6-TRIM34,* for differentiating patients suffering from a severe RSV infection from patients that have a mild or moderate RSV.

In a preferred embodiment, a lower level of methylation of the genes *ZBTB38* and/or *TRIM6-TRIM34,* as compared with a pre-established reference level of methylation of the genes *ZBTB38* and/or *TRIM6-TRIM34* measured in patients that have a mild or moderate infection with RSV, is an indication that the subject is suffering from a severe infection with RSV and/or that the patient has a poor prognosis.

In a preferred embodiment, the methylation status of the gene is determined in at least a CpG site of the gene.

In a preferred embodiment, the methylation status of the gene *ZBTB38* is determined in at least a CpG site located between the chromosomal positions chr3:141102599-141107482 and/or wherein the methylation status of the gene *TRIM6-TRIM34* is determined in at least a CpG site located between the chromosomal positions chr11:5616177-5618408 according to the human reference genome hg19.

In a preferred embodiment, the methylation status of the gene *ZBTB38* is assessed in at least a CpG site selected from the group consisting of: cg25395158, cg23967605, cg00527195, cg03183447, cg17356452, cg10394922, cg02936049, cg21380154, cg03848978, cg11421029, cg06820102, cg04352534, cg13544811, cg13318410, cg04011423 and/or cg23371833; and/or the methylation status of the gene *TRIM6-TRIM34* is assessed in at least a CpG site selected from the group consisting of: cg14304349, cg15137954, cg22133704, cg00104484, cg00827519, cg05391002, cg12707994, cg15742496, cg16082503, cg17706456 and/or cg19872996.

In a preferred embodiment, the biological sample is selected from blood, serum, plasma or saliva, most preferably saliva.

For the purpose of the present invention the following terms are defined:
- "CpG site": The CpG sites refers to dinucleotides formed by a cytosine followed by a guanine. Cytosines in CpG dinucleotide can be methylated to form 5-methylcytosines.
- "CpG island" (CGI): CpG islands (or CG islands) are regions with a high frequency of CpG sites. The usual formal definition is a region with at least 200 bp and a GC percentage greater than 50%.
- "Beta-value": The beta-value provides a quantitative measure of methylation at a specific locus. It is calculated as the ratio of methylated signal intensity to the total signal intensity (M/M+U) at a specific cytosine site. Its value ranges from 0 (unmethylated) to 1 (fully methylated).
- "Delta-Beta": absolute difference in mean beta-value between the two groups of comparison, in this case patients with a severe RSV infection vs those who have a mild or moderate infection with RSV.
- "Bisulfite treatment": Treatment of DNA with sodium bisulfite converts unmethylated cytosine to uracil, which is subsequently converted to thymine during PCR amplification, while methylcytosine remains unchanged, as cytosine.
- "Promoter region": The promoter region of the gene comprises the sequences of the 5'UTR and the first exon of the gene.
- As used herein, the term "methylation" will be understood to mean the presence of a methyl group added by the action of a DNA methyl transferase enzyme to a cytosine base followed by a guanine (CpG) nucleotide of a nucleic acid.
- Accordingly, the term, "methylation status" as used herein refers to the presence or absence of methylation in a specific nucleic acid region.
- The expression "lower level of methylation" refers to a decrease in the relative amount of methylation of a nucleic acid, as compared with the subject used as control that, in this case, are patients that have a mild or moderate infection with RSV. Thus, in the present disclosure, the "lower level of methylation" is generally determined with reference to a baseline level represented by the methylation status of a given genomic region in a sample obtained from control subjects. For example, "lower level of methylation" may be at least 2% lower than the baseline level of methylation, for example at least 5% lower than the baseline level of methylation, or at least 10% lower than the baseline level of methylation, or at least 15% lower than the baseline level of methylation, or at least 20% lower than the baseline level of methylation, or at least 25% lower than the baseline level of methylation, or at least 30% lower than the baseline level of methylation, or at least 40% lower than the baseline level of methylation, or at least 50% lower than the baseline level of methylation, or at least 60% lower than the baseline level of methylation, or at least 70% lower than the baseline level of methylation, or at least 80% lower than the baseline level of methylation, or at least 90% lower than the baseline level of methylation.
- Severe/mild/moderate infection: Three different characteristics are considered. For the severe infection: admission to ICU, need for mechanical ventilation (invasive respiratory support) and high Resvinet* score (>15). On the contrary, mild/moderate patients are a more heterogeneous category who were hospitalised but not admitted to ICU, nor needed respiratory support. Their Resvinet score is less than 8. *Resvinet score is a tool used to assess the severity of RSV infection in infants and young children. It is calculated by considering 7 different clinical parameters, each one scored from 0 to 3.
- The term "comprising" means "including", but not limited to what follows the term "comprising". Thus, the use of the term "comprising" indicates that the elements listed are necessary or mandatory, but that other elements are optional and may or may not be present.
- The term "consisting of" means "including" but is limited to what follows the term "consisting of". Thus, the term "consists of" indicates that the elements listed are mandatory, and that other elements may not be present.

### Description of the figures

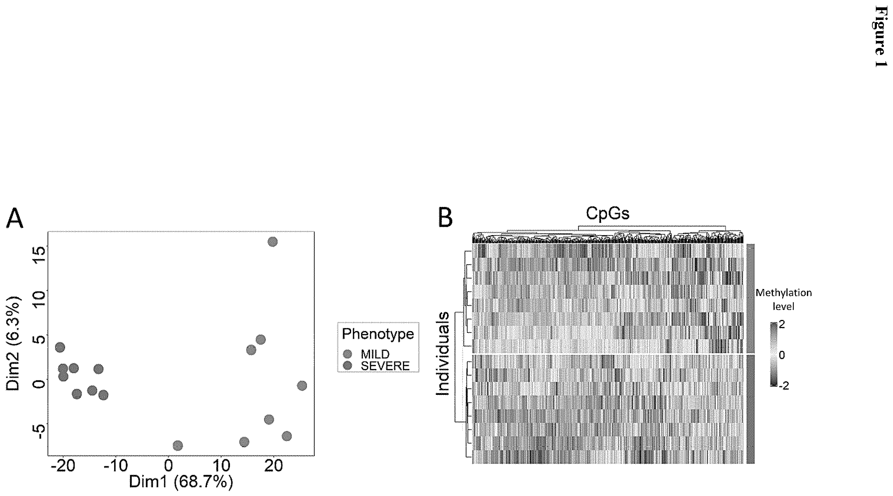

**Figure 1****. Differentially methylated positions distinguish infants with severe infection from those with only mild to moderate symptoms. A.** PCA and **B.** Heatmap of the significant DMPs (nominal P-value < 0.01 and Delta Beta > 0.10) between severe and mild/moderate groups.

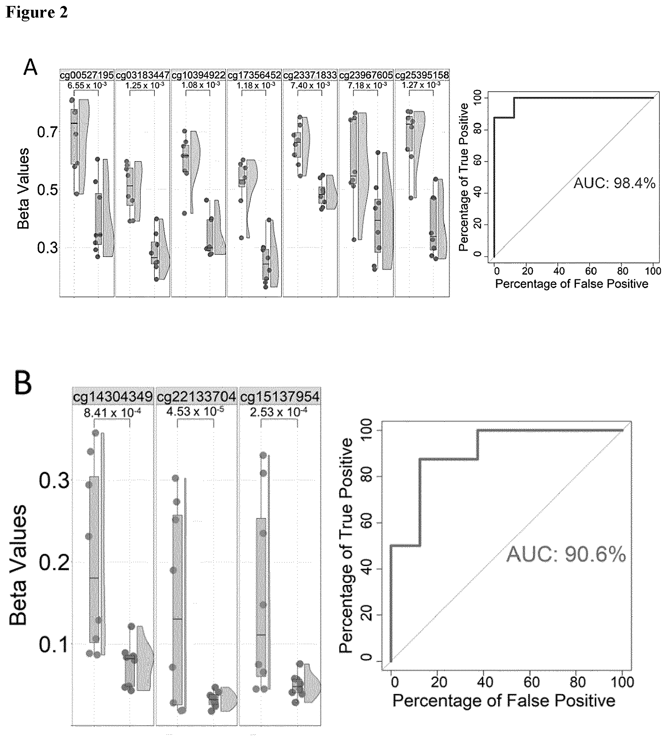

**Figure 2****. Hypomethylation of *ZBTB38* and *TRIM* genes in the severe group.** Boxplot and ROC curve of the cluster of CpGs observed within **A.** *ZBTB38* and **B.** *TRIM6-TRIM34* genes.

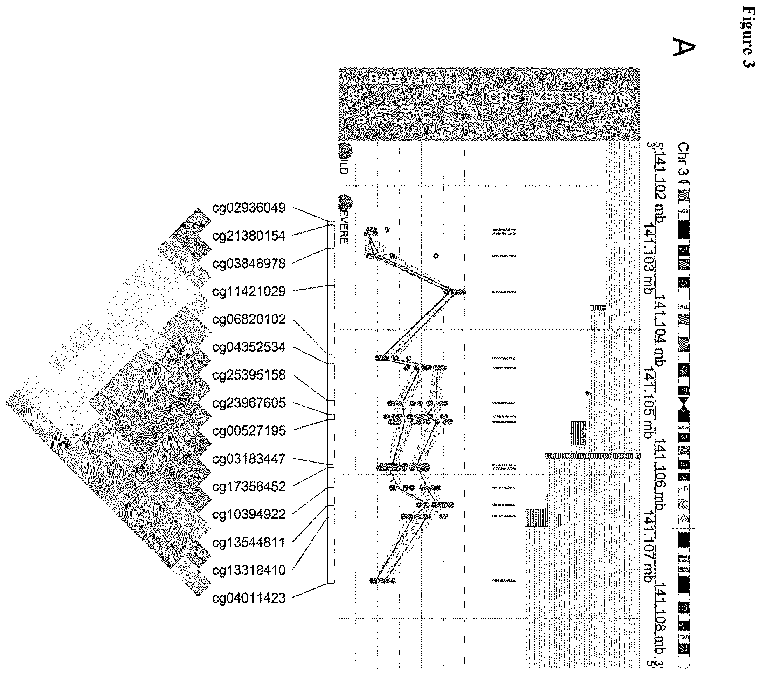

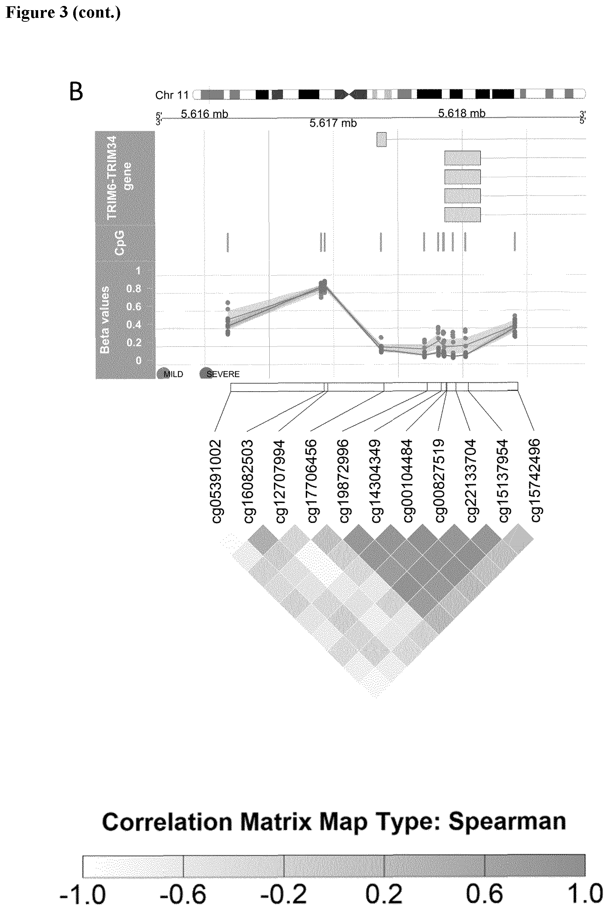

**Figure 3****. Differentially methylated regions distinguish infants with severe infection from those with only mild to moderate symptoms.** Top panel: DMR Plot for the two most significant DMRs within **A.** *ZBTB38* and **B.** *TRIM6-TRIM34* genes. DNA methylation patterns in severe and mild/moderate samples differ for most of the CpGs in the DMRs. In the figure, some β values dots of the CpGs that constitute the DMRs overlap in the two groups having similar values. Bottom panel: pairwise correlation between CpG sites in DMRs in **A.** *ZBTB38* and **B.** *TRIM6-TRIM34* genes.

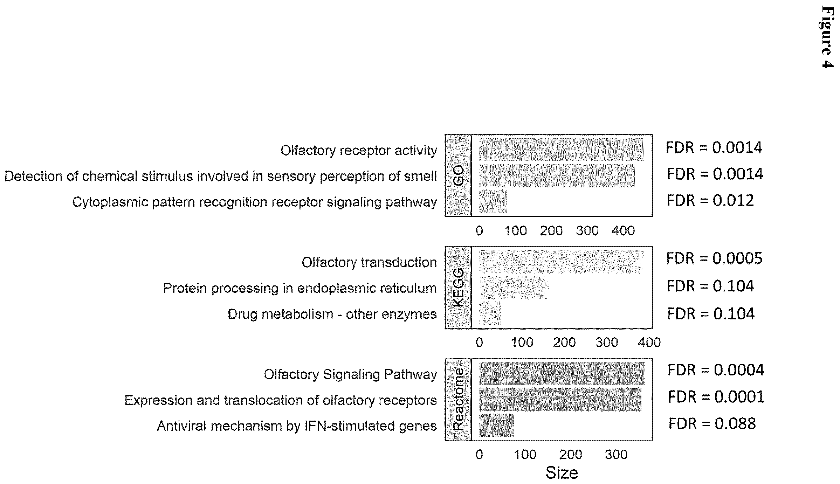

**Figure 4****. Pathways analysis revealed enrichment for pathways associated with olfactory signaling and activation.** Barplot representing the results of the enrichment pathway analysis conducted on all CpGs and specifically on CpGs located within the promoter regions using the *methylglm* approach. The results were obtained using the KEGG, Reactome and GO databases. The size of each bar along the x-axis indicates the number of genes associated with each pathway. Furthermore, false discovery rate (FDR) *P*-values are indicated.

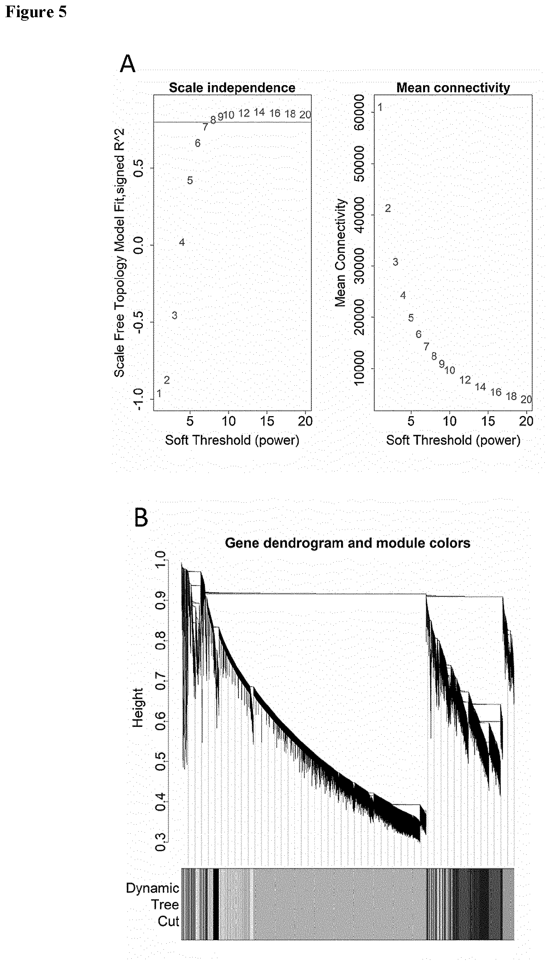

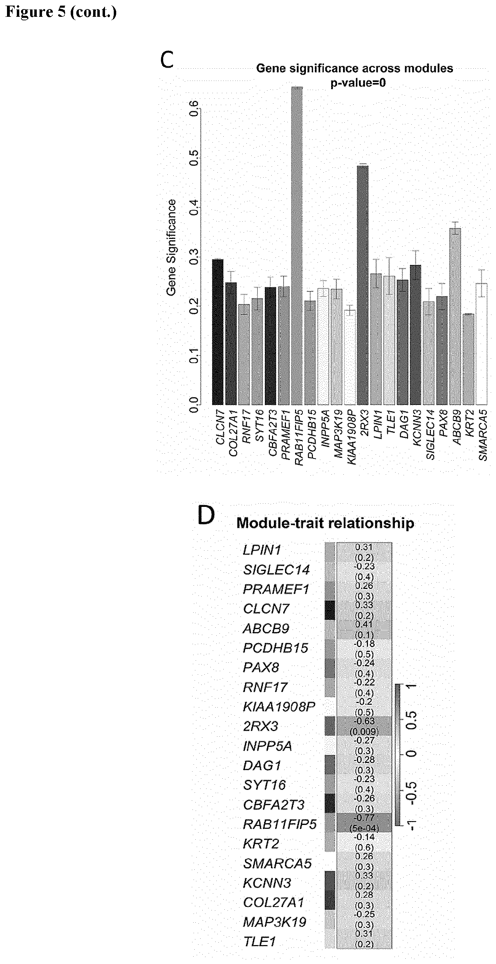

**Figure 5****. Co-methylated modules associated with RSV severity. A.** Selection of the soft-thresholding power involved analyzing plots that depict the correlation between the soft-thresholding powers and two metrics: the scale-free fit index (upper) and the mean connectivity (lower). **B.** Clustering dendrogram of probes, with dissimilarity based on topological overlap, together with assigned module colors. **C.** Barplot displaying the *P*-values from correlation tests between the module eigengenes and RSV severity phenotype. Each module's colors is defined by its hub gene. **D.** Heatmap of Pearson correlation analysis of modules and the clinical trait of RSV severity. Rows represent the 21 module eigengenes and columns represent the phenotype.

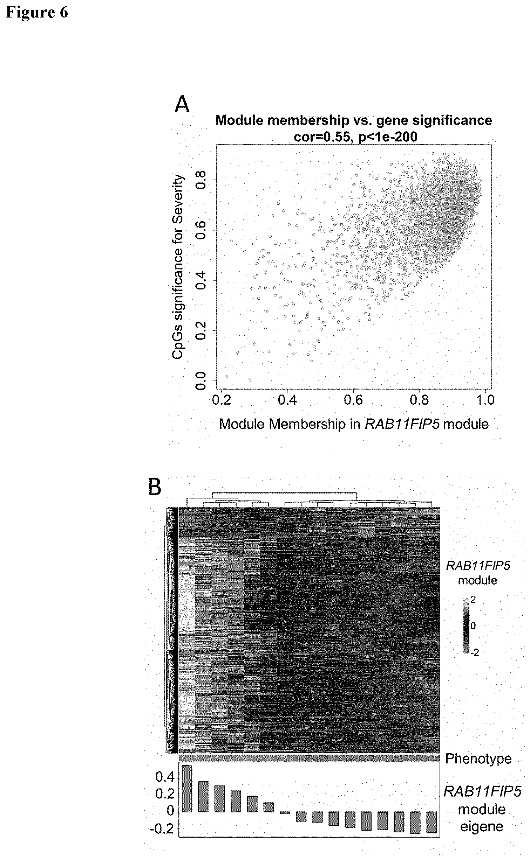

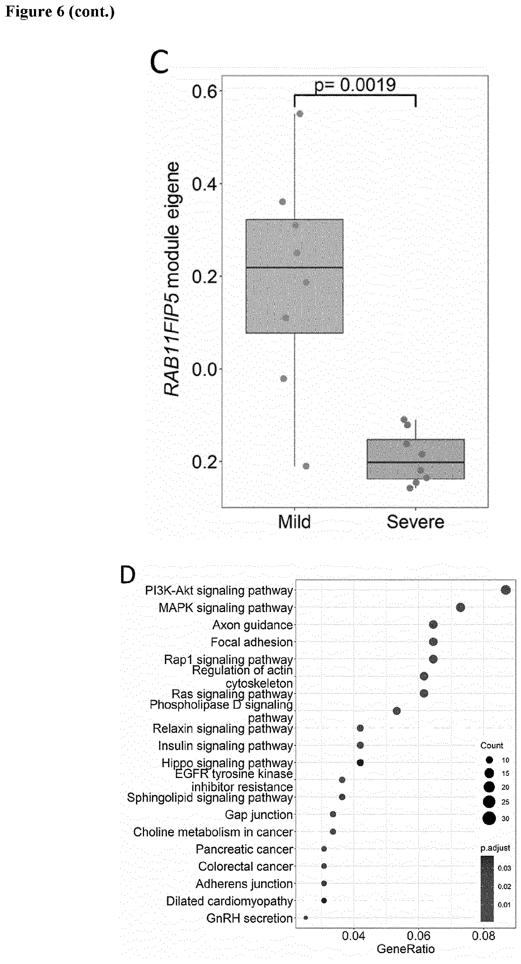

**Figure 6****. Strong negative correlation between the *RAB11FIP5* module and RSV severity. A.** Plot of correlations between gene significance and module membership for the *RAB11FIP5* module. Color-coding is equivalent to module names. **B.** Heatmap displaying the β-values of the CpGs belonging to the *RAB11FIP5* module. Additionally, the eigengene values of the samples are also shown. **C.** Boxplot showing the variations in eigengene values of the samples from the *RAB11FIP5* module between the severe and mild/moderate groups. **D.** Dotplot showcasing the top KEGG pathways with a FDR < 0.01, associated with the *RAB11FIP5* module's genes. The size of each dot on the x-axis represents the number of genes associated with each pathway. Additionally, the color of each dot corresponds to the respective FDR P-values linked to the pathways.

### Detailed description of the invention

The present invention is illustrated by the Examples set below without the intention of limiting its scope of protection.

### Example 1. Material and methods

### Example 1.1. Study participant details

Sixteen patients with RSV infection were chosen from a cohort that was prospectively enrolled in an observational study conducted in Spain, facilitated by a national hospital-based research network for pediatric respiratory research called GENDRES (Genetics, Vitamin D and Respiratory Infections Research Network - www.gendres.org). The RSV infection was confirmed by polymerase chain reaction (PCR) upon presentation to hospital. A saliva ORAGENE sample was collected from each participant during the acute phase of RSV infection, typically within seven days from the onset of symptoms.

The study involving human participants was reviewed and approved by the Ethics Committee of Clinical Investigation of Galicia (CEIC 2016/484). Written informed consent to participate in this study was provided by the participants' legal guardian/next of kin.

**Table 1** presents the clinical history and characteristics of the study subjects. The clinical severity of the disease was determined based on the need for intensive care, invasive respiratory support, and the ReSViNET Score. ReSViNET Score, used as a parameter of severity classification, is a clinical scoring system used to assess patients' severity of acute respiratory infections (ARI). It considers seven parameters: feeding intolerance, medical intervention, respiratory difficulty, respiratory frequency, apnea, general condition, and fever. Each parameter is assigned a value ranging from 0 to 3, resulting in a total score of 20 point.

**Table 1**

| | | **Mild/moderate (*n* = 8)** | **Severe (*n* = 8)** | ***P*-value** | **adjusted *P*-value** |
|---|---|---|---|---|---|
| **Age in years (mean)*** | | 1.06 [0.67] | 0.18 [0.11] | 2.74×10⁻⁰³ | 8.23e⁻⁰³ |
| **Sex** | | | | 0.31 | 0.43 |
| | **Male** | 6 (75%) | 3 (37%) | | |
| | **Female** | 2 (25%) | 5 (63%) | | |
| **Self-reported ancestry** | | | | 0.47 | 0.58 |
| | **South Europe** | 8 (100%) | 6 (75%) | | |
| | **North Africa** | - | 2 (25%) | | |
| **Personal Medical History** | | | | | |
| | **Previous Medical Episode** | 6 (75%) | 2 (25%) | 0.13 | 0.25 |
| | **Previous Episode of Bronquiolitis** | 5 (63%) | - | 0.03 | 0.06 |
| **Fever** | | 5 (63%) | 4 (50%) | 1 | 1 |
| **Oxygen need** | | 4 (50%) | 8 (100%) | 0.08 | 0.16 |
| **ReSViNET Score*** | | 7.60 [2.33] | 15.86 [1.35] | 2.77×10⁻⁰⁶ | 4.16×10⁻⁰⁵ |
| **Respiratory support** | | | | | |
| | **Mechanical*** | - | 8 (100%) | 1.55×10⁻⁰⁴ | 5.83×10⁻⁰⁴ |
| **Suspected bacterial Infection** | | 3 (37%) | 4 (50%) | 0.24 | 0.40 |
| **PICU*** | | - | 8 (100%) | 1.55×10⁻⁰⁴ | 5.83×10⁻⁰⁴ |
| **Antibiotic** | | 4 (50%) | 7 (88%) | 0.28 | 0.42 |
| **Corticosteroids** | | 5 (63%) | 3 (37%) | 0.62 | 0.66 |
| **Salbutamol** | | 5 (63%) | 3 (37%) | 0.62 | 0.66 |

| | | | | | |
|---|---|---|---|---|---|
| *Clinical characteristics of study participants. Wilcoxon and t-test were used for comparison. *Indicates the variables with a statistically significant difference (adjusted P-value) between mild*/*moderate vs. severe group.* | | | | | |

The RSV cases come from a cohort of more than 3000 thousand patients with over half of them being infected by RSV. To select severe RSV cases, children with well-defined severity such as acute bronchiolitis (Lower Respiratory Tract Infection [LRTI]), who have the highest ReSViNET score (>15), require mechanical ventilation, and spend the longest time in the pediatric intensive care unit (PICU), were chosen. The moderate/mild group was formed by selecting children who only require a few days of hospitalization (less than 5), do not require any kind of respiratory support, and have a ReSViNET score below 8. This group of children was diagnosed with both LRTI and Upper Respiratory Tract Infections (URTI).

### Example 1.2. Sample processing

DNA was isolated from saliva samples following the prepIT-L2L (DNAgenoTEK) protocol. Buccal swabs were incubated at 50°C in a water incubator for at least one hour. The saliva sample was then transferred to a microcentrifuge tube and mixed with a 1/25 volume of PT-L2P. The mixture was vortexed and incubated on ice. After centrifugation, the supernatant was removed, and ethanol was added to allow DNA precipitation. Subsequently, an ethanol wash was performed subsequently, and the DNA pellet was then dissolved into TE solution. After isolation, DNA sample concentration and quality were checked using common Nanodrop measurements and Qubit. Because DNA in saliva samples or buccal swabs tends to be more degraded than blood DNA, further DNA degradation control was performed to ensure the optimal DNA concentration for the subsequent steps. The whole Illumina methylation EPIC BeadChip experiment was carried out in the Genómica e Investigación Oncológica (GENYO) center (Granada; Spain). The epigenome data were delivered to our group in Santiago de Compostela for subsequent bioinformatic and statistical analysis.

The appropriate volume and concentrated DNA were submitted to bisulphite conversion, a gold standard technique that allows the deamination of non-methylated cytosines into uracil and maintains the methylated cytosine unaffected. After this treatment, the subsequent amplification recognizes uracil as thymine, and methylated cytosines as cytosines. The level of DNA methylation is then calculated by quantitative genotyping of C/T SNPs. After hybridization to the Illumina methylation EPIC BeadChip platform, the fluorescence intensity resulting from the incorporation of a nucleotide in the probe is translated into a level of DNA methylation (DNAm) for each CpGs site. DNAm can be defined as Beta- or M-values. Beta-values range between 0 and 1, where 0 represents non-methylation, and 1 represents 100% methylation. Beta-values are used for graphics due to their easy interpretation. M-values, on the other hand, are the logit-transformed Beta-values; homoscedasticity is generally assumed for these values, and they are preferentially used for linear statistical analysis.

### Example 1.3. Statistical Analysis

### Example 1.3.1. Methylation Data Analysis

Before calculating the Beta- and M-values, raw IDAT data, were subjected to different filtering processes and normalization steps to obtain high-quality data suitable for downstream analysis.

In the analysis of DNAm data derived from 'bulk' tissues such as blood or saliva, it is crucial to estimate the composition of different cell types within the tissue. Since the measured of DNAm in 'bulk' tissues represents an average of the methylation levels from multiple cell types, there is a risk that the overall DNAm level is predominantly influenced by specific cell types. Moreover, when employing saliva as a biomarker identification tool, it is important to consider its extreme cellular heterogeneity. It has been demonstrated that buccal swabs, used to collect saliva samples, contain a higher proportion of epithelial cells than saliva, and this proportion is higher in children when compared to adults. Therefore, estimating the cell composition is an essential step in this type of analysis. In this analysis, we used the *Epidish* R package to estimate cell composition of buccal swab samples. The *hepidish* function of the *Epidish* R package, allowing the use of two different DNAm references, was applied to the betas values to specifically estimate epithelial cells, fibroblasts, and total immune cells (neutrophils, eosinophils, monocytes, CD4+ and CD8+ T cells, B cells, and natural killer [NK] cells) (47, 48).

### Example 1.3.2. Identification of RSV severity-associated differential methylation

The *Limma* package was used to identify DMPs, employing a model adjusted for age, sex, and cell estimation. To account for the age variable, we included it as a covariate in the linear model formula. This allowed us to estimate the effect of other variables while controlling for the effect of age. Specifically, we created a design matrix including age as a covariate and fitted a linear model using the *ImFit*() function. The previous episode of bronchiolitis was taken into account in the statistical analysis to evaluate the effect of this clinical factor, which appeared in five out of eight mild/moderate individuals. The threshold for identifying DMPs was set at an adjusted *P*-value (False Discovery Rate or FDR) of < 0.01 and an absolute difference in DNAm between groups (Delta Beta) of > 0.10. Receiver operating characteristic (ROC) curve analyses were conducted to evaluate the diagnostic efficacy of the most significant candidate DMPs, and the determined area under the curve (AUC) was used to assess prediction accuracy.

To identify DMRs, the *DMRcate* package was utilized with default parameters. The package employs Gaussian kernel smoothing to detect patterns of differential methylation independent of genomic annotation. In this study, a bandwidth (λ) of 1000 base pairs and a scaling factor (C) of 2 were used. Despite working with a sparse set of sites, the aim was to identify regions with multiple probe clusters (minimum of 3 CpGs per region), exhibiting substantial effect sizes and a minimum smoothed FDR < 0.01.

### Example 1.3.3. Pathways analysis

For gene set enrichment analysis, the *methylGSA* package was used, using methylglm a function.

This function extends GOglm by incorporating gene length as a covariate to adjust length bias in DNAm based on the number of CpGs. Pathway enrichment analysis were customized in two ways: considering all CpGs regardless of their gene group and focusing only on CpGs within the promoters based on the annotation in the "IlluminaHumanMethylationEPICanno.ilm10b4.hg19regions" (TSS200, TSS1500, 5'UTR, 1stExon).

### Example 1.3.4. Weighted Gene Correlation Network Analysis (WGCNA)

WGCNA package was used to construct a signed weighted correlation network, using the top 20% of positions with the highest median variance (approximately 120,000). To identify the gene module with the strongest association to RSV severity, a series of steps were performed. Firstly, the soft threshold power was selected based on the standard scale-free networks, and a power function was used to calculate all differential CpGs. The adjacency matrix was then transformed into a Topological Overlap Matrix (TOM), and the corresponding dissimilarity matrix (1-TOM) was calculated. The dynamic tree cut method was employed to hierarchically cluster CpGs and identify the module, with a minimum module size of 30, medium sensitivity for cluster splitting, and a dendrogram cut height threshold of 0.25 for module merging as module detection parameters. Unassigned CpG-sites were clustered in the "grey" module, which was not considered for further analyses. Gene Significance (GS) was computed to detect statistically significant associations between modules and phenotype, and module membership (MM) was also calculated as a measure of intramodular connectivity by correlating (using Pearson correlation) the methylation profile with the eigengene of a given module. The correlation between GS and MM was investigated, and the average absolute gene significance for all CpGs within a module was determined to identify the most important modules. Modules were named by their most significant hub genes. Furthermore, hub genes of RSV severity-associated modules were extracted, and an over-representation analysis of the most important module related to the trait was performed using the *Clusterprofiler* R package and the Kyoto Encyclopedia of Genes and Genomes (KEGG) database as a reference to investigate its biological significance. The R statistical package (v. 4.2.2) was used for all the statistical analyses.

### Example 2. Results

### Example 2.1. Clinical characteristics of patients

**Table 1** describes the clinical characteristics of patients. Most of these characteristics exhibit similarities between the two severity groups. Clinical severity factors, such as the ReSViNET score, the PICU admission, and the requirement for mechanical ventilation, are found to be significant among the two groups, as expected. Another statistically significant factor was age, with infants in the severe group being younger than those with a mild infection. To investigate the association between the observed severity in the subjects and their "biological age," we employed age predictors. Specifically, we utilized the Pediatric-Buccal-Epigenetic (PedBE) clock, an epigenetic age estimator designed to pediatric buccal swab samples. Through this analysis, we found a strong correlation (rho, ρ = 0.96; *P*-value < 0.0001) between DNAm (DNA methylation) age and chronological age. Consequently, we did not observe statistically significant differences in predicted biological age between the mild/moderate and severe groups. To mitigate confounding effects on the results, age has been considered as a covariate in all analyses.

### Example 2.2. Identification of Differentially Methylated Positions and Regions within ZBTB38 and TRIM6-TRIM34 genes

The estimation of cell composition did not reveal any significant differences between the two cohorts. Therefore, to minimize potential confounding factors, age was the only significant variable adjusted in the linear model. Out of the 803,527 total DNA probes analyzed, we identified 461 Differentially Methylated Positions (DMPs) (assuming a nominal P-value < 0.01, and an absolute difference in methylation level [Delta Beta] > 0.10) between infants with a severe infection and those with only mild/moderate symptoms. Among these, the majority (n = 339, 73.5%) exhibited hypomethylation in the severe group when compared to the mild/moderate group, while the remaining (n = 122, 26.5%) showed hypermethylation. These DMPs enabled the differentiation of patient's methylation profiles into two distinct groups in the Principal Component Analysis (PCA) and heatmap (**Figure 1A** and **1B**). The main differentiation in the PCA comes from the first principal component (PC1), that accounts for 68.7% of the variation.

Notably, we also identified clusters of DMPs within promoter regions and belonging to the same gene. Specifically, we found seven positions in the 5'UTR region of the *ZBTB38* gene **(Table 2** and **Figure 2A****)** and three positions within the TSS200 region of *TRIM6-TRIM34* genes **(Table 2** and **Figure 2B**). Adjusting the model for both age and bronchiolitis, we observed a decrease in the number of DMPs (*n* = 129; nominal *P*-value < 0.01). However, despite this reduction, the top DMPs remained consistent, particularly those located within the *ZBTB38* (four positions) and the *TRIM6-TRIM34* (three positions) genes; **Table 2.** Additionally, removing patients who have experienced previous episodes of bronchiolitis from the statistical analysis resulted in a slightly different but even larger group of DMPs (n = 465). However, what is more relevant again is the remarkable consistency in the top DMPs and DMRs, pointing to *ZBTB38* (with two positions) and *TRIM6-TRIM34* (with five positions); **Table 2.** This compelling consistency implies that previous episodes of bronchiolitis might not exert a widespread impact on DNA methylation across the entire moderate cohort, reinforcing the robustness of the findings.

**Table 2**

| **CpGs** | **Position** | **Gene Name** | **Gene Group** | **Delta Beta** | ***P*-value** |
|---|---|---|---|---|---|
| cg14304349*^{1,2} | chr11: 5617812 | *TRIM6-TRIM34* | TSS200;5'UTR | 0.13 | 8.41×10⁻⁰⁴ |
| cg15137954*^{1,2} | chr11: 5618023 | *TRIM6-TRIM34* | 1stExon; 5'UTR | 0.11 | 4.53 ×10⁻⁰⁵ |
| cg22133704*^{1,2} | chr11: 5617926 | *TRIM6-TRIM34* | 1stExon; 5'UTR | 0.11 | 2.53 ×10⁻⁰⁴ |
| cg23371833 | chr3: 141133836 | *ZBTB38* | 5'UTR | 0.17 | 7.40 ×10⁻⁰³ |
| cg23967605 | chr3: 141105198 | *ZBTB38* | 5'UTR | 0.20 | 7.18 ×10⁻⁰³ |
| cg03183447*^{1,2} | chr3: 141105876 | *ZBTB38* | 5'UTR | 0.22 | 1.25 ×10⁻⁰³ |
| cg10394922*^{1,2} | chr3: 141106189 | *ZBTB38* | 5'UTR | 0.26 | 1.08 ×10⁻⁰³ |
| cg17356452*' | chr3: 141105920 | *ZBTB38* | 5'UTR | 0.27 | 1.18 ×10⁻⁰³ |
| cg00527195 | chr3: 141105273 | *ZBTB38* | 5'UTR | 0.29 | 6.55 ×10⁻⁰³ |
| cg25395158*¹ | chr3: 141105014 | *ZBTB38* | 5'UTR | 0.31 | 1.27 ×10⁻⁰³ |

| | | | | | |
|---|---|---|---|---|---|
| *Cluster of Differentially Methylated Positions (DMPs) within TRIM6-TRIM34 and ZBTB38 genes. Delta Beta refers to the comparison mild*/*moderate vs. severe patient's groups. * IDMPs after adjusting the model for age and previous episodes of bronchiolitis. *2DMPs when excluding children with previous episodes of bronchiolitis.* | | | | | |

To assess the potential predictive value of DMPs from these genes (*ZBTB38* and *TRIM6-TRIM34)* as biomarkers of RSV severity, we performed ROC analysis with AUC calculations. The total ROC curve for the seven DMPs in the *ZBTB38* gene yielded an AUC of 0.984 (0.941-1.000), while the ROC curve for the three DMPS within the *TRIM6-TRIM34* region returned an AUC of 0.906 (0.756-1.000).

Furthermore, we utilized *DMRcate* to identify Differentially Methylated Regions (DMRs) comprising at least three neighboring CpG sites associated with RSV severity in the same direction. This approach revealed eight regions with decreasing methylation and twelve regions with increasing methylation **(Table 3**). This analysis revealed two highly statistically significant regions **(Table 3**). The most significant one (FDR = 2.83 × 10⁻²⁴) overlaps the *TRIM6-TRIM34* genes, and it comprises 11 highly correlated CpGs (**Figure 3A**)**.** The second most significant region (FDR of 1.93 × 10⁻¹¹) overlaps with the *ZBTB38* gene, and it exhibits 15 CpGs, and it contains a core of highly correlated CpGs (**Figure 3B**).

**Table 3**

| **Position** | **Overlapping Genes** | ***n* CpGs** | **Delta Beta** | **FDR** |
|---|---|---|---|---|
| chr2:47261254-47262234 | *TTC7A* | 3 | 0.15 | 3.19×10⁻⁰⁶ |
| chr3:192289245-192289293 | *FGF12* | 3 | 0.12 | 9.27×10⁻⁰⁴ |
| chr5:178576749-178577542 | *ADAMTS2* | 3 | 0.12 | 3.70×10⁻⁰³ |
| chr19:41385865-41386507 | *CTC-490E21.12, CYP2A7* | 3 | 0.16 | 1.05×10⁻⁰⁵ |
| chr1:149162219-149162518 | | 4 | 0.12 | 4.90×10⁻⁰³ |
| chr2:91932642-91933195 | *AC027612.4* | 4 | 0.12 | 5.96×10⁻⁰³ |
| chr17:27456682-27456844 | *MYO18A* | 4 | 0.16 | 2.06×10⁻⁰⁴ |
| chr4:674399-675936 | *MYL5, MFSD7* | 5 | -0.16 | 2.58×10⁻⁰⁴ |
| chr15:77816887-77818281 | *RP11-307C19.1* | 5 | -0.11 | 2.09×10⁻⁰⁴ |
| chr2:113990230-113995456 | *PAX8-AS1, PAX8* | 16 | 0.11 | 3.91×10⁻⁰³ |
| **chr11:5616177-5618408** | ***TRIM6-TRIM34, HBG2, AC015691.13*** | **11** | **-0.11** | **2.83×10⁻²⁴** |
| chr2:162099304-162101506 | *AC009299.3, AC009299.2* | 7 | 0.12 | 1.16×10⁻⁰⁷ |
| chr11:60414241-60414918 | *LINC00301* | 7 | 0.10 | 6.38×10⁻⁰⁶ |
| chr1:149144161-149148312 | *RNU1-114P* | 18 | 0.12 | 2.18×10⁻⁰⁸ |
| **chr3:141102599-141107482** | ***ZBTB38*** | **15** | **-0.14** | **1.93×10⁻¹¹** |
| chr16:49732224-49733377 | *ZNF423* | 6 | -0.10 | 1.25×10⁻⁰⁴ |

| | | | | |
|---|---|---|---|---|
| *Differentially Methylated Regions (DMRs) associated with RSV severity. The top statistically significant regions are marked in bold. Delta Beta refers to the comparison mild*/*moderate vs. severe patient's groups. FDR: means here 'minimum smoother FDR*'. | | | | |

### Example 2.3. Pathways analysis

We next investigated gene sets from Reactome, Gene Ontology (GO), and KEGG pathways, by considering *i*) all CpGs, and *ii*) only CpGs within promoter regions. Remarkably, for both analyses, significant enrichment was observed in three Reactome pathways, three GO biological processes, and one KEGG pathway (**Figure 4**). Notably, all three enriched pathway analyses included olfactory signaling pathways, such as transduction and receptor activity.

### Example 2.4. Negative correlation between co-methylated modules and the RSV severity

Co-methylated modules were constructed using the *WGCNA* R package with the top 20% of probes exhibiting the highest mean-variance (*n* = 114,806). After testing a set of candidate powers, a soft-thresholding power of seven was selected based on the criterion of scale-free topology (**Figure 5A**). The analysis revealed 32 modules, each containing at least 30 co-methylated genes, labeled by a color name (**Figure 5B**). Applying a cut height threshold of 0.25 for module merging resulted in the identification of 21 modules. The module-trait relationships were tested by calculating the correlation between the modules and the severity phenotype (**Figure 5C**), resulting in two significantly associated modules: *RAB11FIP5* (green) and *2RX3* (magenta) (**Figure 5D**). However, after adjusting for multiple testing, only the *RAB11FIP5* module survived with an FDR P-value < 0.011. We found a strong correlation between GS and MM for this module, indicating that CpGs associated with RSV severity are also core elements of that module (**Figure 6A**).

A strong negative correlation (R = -0.77) between the *RAB11FIP5* module and the trait was observed, indicating a hypomethylation state in the severely ill group compared to the mild/moderate group. This finding is consistent with the results obtained from DMPs and DMRs, where lower levels of methylation were observed in the severe group compared to the mild/moderate. Heatmap and sample eigengenes plots strongly support these results, demonstrating an overall lower methylation value in the cohort of patients with severe symptoms (**Figure 6B** and **6C**). Within the ***RAB11FIP5*** module, 153 CpGs of the previously identified 461 DMPs (33%) were included, of which 97 (21%) are considered hub CpGs (GS < -0.7 and MM > 0.8).

We identified a total of 1135 hub CpGs (hCpGs) within the *RAB11FIP5* module **(Table** S2). Upon examining the cluster of these hCpGs falling within specific (hub) genes, six hCpGs were observed within the *CUX1* and the *ANK3* genes; four within the *ZBTB38,* four in the body region of the *SSBP3,* and four within *GLI3, FOXP1,* and *AFF3.* The genes associated with the most significant module hCpGs are primarily involved in pathways related to PI3K-Akt, MAPK, Rap1, and Ras signaling pathways. (**Figure 6D**).

## Claims

1. *In vitro* method for the prognosis of a Respiratory Syncytial Virus infection, which comprises determining the methylation status of a gene selected from the group consisting of: *ZBTB38* and/or *TRIM6-TRIM34,* in a biological sample obtained from the patient.

2. *In vitro* method, according to claim 1, for differentiating patients suffering from a severe Respiratory Syncytial Virus infection from patients that have a mild or moderate Respiratory Syncytial Virus infection, which comprises determining the methylation status of the gene selected from the group consisting of: *ZBTB38* and/or *TRIM6-TRIM34,* in a biological sample obtained from the patient.

3. *In vitro* use of the methylation status of a gene selected from the group consisting of: *ZBTB38* and/or *TRIM6-TRIM34,* or of a kit comprising reagents for determining the methylation status of a gene selected from the group consisting of: *ZBTB38* and/or *TRIM6-TRIM34,* for the prognosis of a Respiratory Syncytial Virus infection.

4. *In vitro* use, according to claim 3, of the methylation status of a gene selected from the group consisting of: *ZBTB38* and/or *TRIM6-TRIM34,* or of a kit comprising reagents for determining the methylation status of a gene selected from the group consisting of: *ZBTB38* and/or *TRIM6-TRIM34,* for differentiating patients suffering from a severe Respiratory Syncytial Virus infection from patients that have a mild or moderate Respiratory Syncytial Virus infection.

5. *In vitro* method, or *in vitro* use, according to any of the previous claims, wherein a lower level of methylation of the genes *ZBTB38* and/or *TRIM6-TRIM34,* as compared with a pre-established reference level of methylation of the genes *ZBTB38* and/or *TRIM6-TRIM34* measured in patients that have a mild or moderate infection with Respiratory Syncytial Virus, is an indication that the subject is suffering from a severe infection with Respiratory Syncytial Virus and/or that the patient has a poor prognosis.

6. *In vitro* method, or *in vitro* use, according to any of the previous claims, wherein the methylation status of the gene is determined in at least a CpG site of the gene.

7. *In vitro* method, or *in vitro* use, according to any of the previous claims, wherein the methylation status of the gene *ZBTB38* is determined in at least a CpG site located between the chromosomal positions chr3:141102599-141107482 and/or wherein the methylation status of the gene *TRIM6-TRIM34* is determined in at least a CpG site located between the chromosomal positions chr11:5616177-5618408 according to the human reference genome hg19.

8. *In vitro* method or *in vitro* use, according to any of the previous claims, wherein the methylation status of the gene *ZBTB38* is assessed in at least a CpG site selected from the group consisting of: cg25395158, cg23967605, cg00527195, cg03183447, cg17356452, cg10394922, cg02936049, cg21380154, cg03848978, cg11421029, cg06820102, cg04352534, cg13544811, cg13318410, cg04011423 and/or cg23371833; and/or the methylation status of the gene *TRIM6-TRIM34* is assessed in at least a CpG site selected from the group consisting of: cg14304349, cg15137954, cg22133704, cg00104484, cg00827519, cg05391002, cg12707994, cg15742496, cg16082503, cg17706456 and/or cg19872996.

9. *In vitro* method or *in vitro* use, according to any of the previous claims, wherein the biological sample is selected from blood, serum, plasma or saliva.

10. *In vitro* method or *in vitro* use, according to any of the previous claims, wherein the biological sample is saliva.
